# EUROPEAN PATENT SPECIFICATION

(11) **EP 0 914 829 B1**
(45) Date of publication and mention of the grant of the patent: **18.06.2008**
(21) Application number: 98907158.4
(22) Date of filing: 11.03.1998
(51) Int. Cl.: A61K 38/22, A61K 38/18

(54) **PREVENTIVES AND/OR REMEDIES FOR MULTIPLE ORGAN FAILURE**
VORBEUGE- UND/ODER HEILMITTEL FÜR DAS MULTIPLE ORGANVERSAGEN
MEDICAMENTS PREVENTIFS ET/OU REMEDES POUR DEFAILLANCE DE MULTIPLES ORGANES

(30) Priority: 11.03.1997 JP 7437297; 30.05.1997 JP 15764597
(43) Date of publication of application: 12.05.1999
(73) Proprietor: Daiichi Sankyo Company, Limited, Chuo-ku Tokyo 103-8426 (JP)
(72) Inventor: ARISAWA, Hirohiko, Kawachi-gun, Tochigi 329-043444 (JP); MASUNAGA, Hiroaki, Shimotsuga-gun, Tochigi 321-0222 (JP); OGAWA, Hiromi, Utsunomiya-shi, Tochigi 320-0056 (JP); HIGASHIO, Kanji, Kawagoe-shi, Saitama 350-0822 (JP)
(74) Representative: Wakerley, Helen Rachael
(86) International application number: PCT/JP1998/000998
(87) International publication number: WO 1998/040096

(56) References cited:
- EP-A- 0 588 477
- EP-A- 0 821 969
- JP-A- 4 049 246
- JP-A- 6 025 273
- JP-A- 6 040 935
- JP-A- 6 040 938
- JP-A- 6 056 692
- JP-A- 6 247 872
- JP-A- 8 176 007
- LEE W M: "Acute liver failure." AMERICAN JOURNAL OF MEDICINE, (1994 JAN 17) 96 (1A) 3S-9S. REF: 49 JOURNAL CODE: 0267200. ISSN: 0002-9343., January 1994 (1994-01), XP0008029885
- TOYOHIRO TAKEHARA et al., "Structure of Hepatic Cell Growth Factor (HGF) and Physiological Effect (in Japanese)", PROTEIN, NUCLEIC ACID AND ENZYME, 1991, Vol. 36, No. 7, pp. 265-274, XP002921869
- HUMES H.D. et al., "Renal Tubule Cell Repair Following Acute Renal Injury", MINER. ELECTROLYTE METAB., 1995, Vol. 21, pp. 353-365, XP002921870
- NOBUYUKI SHIMA et al., "Structure of Tumor Cell Damage Factor (F-TCF) and Various Bioactivity (in Japanese)", XP002916991

## Description

### Technical field

Disclosed is an agent for preventing and/or treating multiple organ failure.

Development from burn, disseminated intravascular coagulation (DIC), circulatory failure, hemorrhagic shock, infectious disease, acute pancreatitis, ischemic disorder, hepatorenal syndrome, gastrointestinal hemorrhage, nutritional metabolic failure, terminal cancer, acquired immunodeficiency syndrome (AIDS), deterioration of systemic conditions due to radiation affection and cachexia etc. to multiple organ failure can be prevented or treated by the present invention.

### Background technology

Onset or exacerbation of multiple organ failure can be classified into the following 3 categories with respect to mechanism: (1) Parallel induction of several organ disorders due to the same factor, (2) Induction of a specific organ dysfunction due to disorder of an organ; and (3) Participation of an iatrogenic factor. Excessive insults due to severe trauma or major surgeries, infectious diseases, shock etc. directly or through various kinds of mediator participate in the onset or deterioration of multiple organ failure by mechanism (1). In the case of multiple organ failure accompanied with organ disorder due to trauma or primary hepatic insufficiency, participation of mechanism (2) through organ correlation mechanism will largely contribute to the onset or deterioration thereof.. By mechanism (3), medical care carried out during intensive care or care to correspond with an organ disorder may result in the other organ disorder. In patients, these 3 mechanisms participate to the development or deterioration of disorder in a complexed manner. The prognosis of patients of multiple organ failure is generally very poor and , in fact, the survival rate is low as 20-30% in spite of a wide variety of corresponding treatment.

### Disclosure of the present invention

Considering the above situations, the present inventors eagerly investigated to look for the use of an agent in the manufacture of a medicament for preventing and/or treating multiple organ failure and found that multiple organ failure caused by burn, disseminated intravascular coagulation (DIC), circulatory failure, hemorrhagic shock, infectious disease, acute pancreatitis, ischemic disorder, hepatorenal syndrome, gastrointestinal hemorrhage, nutritional metabolic failure, terminal cancer, acquired immunodeficiency syndrome (AIDS), deterioration of systemic conditions due to radiation affection and cachexia etc. can be prevented or treated with tumor cytotoxic factor-II (TCF-II) which is a glycoprotein derived from human fibroblastor hepatocyte growth factor (HGF) which is a proteineous substance derived from blood of a patient with fulminating hepatitis. Accordingly, an object of the present invention is to provide the use of an agent in the manufacture of a medicament for preventing and/or treating multiple organ failure comprising TCF-II or HGF as an effective ingredient

The present invention relates to the use of an agent in the manufacture of a medicament for preventing and/or treating multiple organ failure comprising TCF-II or HGF as an effective ingredient.

### Brief description of the drawings

Figure 1 shows the protective effect of TCF-II on endotoxin-induced multiple organ failure in example 4.
Figure 2 shows the protective effect of HGF on endotoxin-induced multiple organ failure in example 5.
Figure 3 shows the protective effect of TCF-II on dimethylnitrosamine-induced multiple organ failure in example 7.
Figure 4 shows the protective effect of TCF-II on thioacetamide intoxication-induced multiple organ failure in example 8.
Figure 5 shows the protective effect of TCF-II on acetaminophen intoxication-induced multiple organ failure in example 8.
Figure 6 shows the protective effect of TCF-II on multiple organ failure caused by mercuric chloride-induced renal insufficiency in example 9.
Figure 7 shows the protective effect of TCF-II on tripsin-induced multiple organ failure in example 10.
Figure 8 shows the protective effect of TCF-II on burn-induced multiple organ failure in example 11.
Figure 9 shows the protective effect of TCF-II on burn-induced multiple organ failure in example 12.
Figure 10 shows the protective effect of HGF on burn-induced multiple organ failure in example 13.

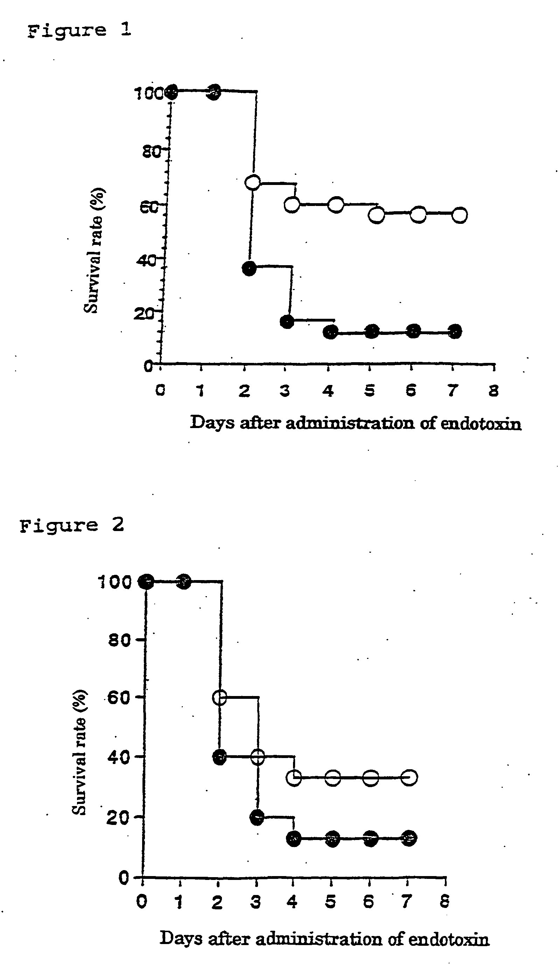

In figure 1-10, ○ represents the TCF-II or HGF treatment group and ● represents the vehicle treatment group.

### Best mode of embodiment for the practice of the present invention

The agent disclosed for preventing and/or treating multiple organ failure can be useful for preventing and/or treating the development from burn, disseminated intravascular coagulation (DIC), circulatory failure, hemorrhagic shock, infectious disease, acute pancreatitis, ischemic disorder, hepatorenal syndrome, gastrointestinal hemorrhage, nutritional metabolic failure, terminal cancer, acquired immunodeficiency syndrome (AIDS), deterioration of systemic conditions due to radiation affection and cachexia etc. These kinds of multiple organ failure will develop by accompanying with burn, surgical operation, administration of chemical substances (including medicine), radiation or other disorder.

TCF-II which is an effective ingredient disclosed was found by the present inventors and a known glycoprotein derived from human fibroblast having the following characteristics:
1) Molecular weight(by SDS electrophoresis)

| | | |
|---|---|---|
| under non-reducing conditions: | 78,000 ± | 2,000 or 74,000 |
| | ± | 2,000 |
| under reducing conditions: | 52,000 ± | 2,000 (common b and A) |
| | 30,000 ± | 2,000 (b and B) |
| | 26,000 ± | 2,000 (b and C) |

2) Isoelectric point: 7.4-8.6

The above TCF-II can be obtained by adsorbing culture medium of human fibroblast on an ion exchange column then purifying the elute by affinity chromatography (WO 90/10651) or by genetic engineering manipulation (WO 92/01053). TCF-II which is an effective ingredient disclosed can be derived from fibroblast or produced by genetic engineering manipulation using microbial or other cell as based on the genetic sequence described in patent application WO 90/10651. Further, TCF-II obtained by genetic engineering manipulation described in WO 92/01053 can be also used. TCF-II with different carbohydrate chain or without carbohydrate chain due to difference of host cell or microbial organism can be also used. However, since carbohydrate chain correlate to metabolic rate in a biological body, one with carbohydrate chain can be preferably used. TCF-II obtained by these methods can be concentrated and purified by usual isolation and purification method. For example, precipitation with organic solvent, salting-out, gel permeation, affinity chromatography using monoclonal antibody, electrophoresis can be exemplified. Purification by affinity chromatography using monoclonal antibody can be carried out using monoclonal antibody described in a Japanese unexamined laid open patent application No.97(1993). The obtained TCF-II can be lyophilized or kept frozen.

Substance having the same activity as TCF-II can be used as the agent. For example, hepatocyte growth factor (HGF; Japanese unexamined laid-open patent application No.22526 (1988)) which is formed by insertion of 5 amino acids into TCF-II protein or purified Scattered Factor (SF; Gherardi and Stocker, Nature, 346, 228 (1990)) can be exemplified.

HGF which is an effective ingredient has an activity proliferating hepatic cell, was isolated from the blood of a patient with fulminating hepatitis and is unknown protein with the following characteristics (Japanese patent No.2564486):
1) Molecular weight (SDS-PAGE); under non-reducing conditions; 76,000-92,000;
2) The activity described above was not deactivated by heating at 56°C for 15minutes, but it was deactivated by heating at 809C for 10 minutes;
3) Digestion with trypsin or chymotrypsin deactivated the above activity; and
4) It show affinity with heparin.

HGF can be obtained by heating plasma at 56 °C for about 15 minutes, taking precipitated fraction at the ammonium sulfate concentration of 1.1-1.2 M, followed by purification using gel permeation and ion exchange chromatography such as DEAE anion exchange chromatography. Alternatively, HGF can be obtained by genetic engineering manipulation using HGF cDNA (BRRC 163, 967-973, 1989 , or Japanese unexamined laid open patent application No.97(1993)).

Documents JP-A-6 247 872 & EP-A-0 612 530 relate to a pharmaceutical composition comprising TCF-II or HGF and JP-A-8 176 007 & EP-B1-0 754 051 and JP-A-4 049 246 & EP-A-0 462 549 relate to medical uses of TCF-II or HGF for the treatment of hypercholesterolemia and for the prevention of progression of hypo-high density-lipoproteinemia and for the treatment or prevention of renal diseases.

Documents EP-A-0 588 477 & EP-A-0 821 969, Lee, W.M.: "Acute Liver Failure" (AMERICAN JOURNAL OF MEDICINE, vol. 96, no. 1A, 3S-9S, January 1994), and Lake, E.W. & Humes, H.D.: "Acute Renal Failure: Directed Therapy to Enhance Renal Tubular Regeneration" (SEMINARS IN NEPHROLOGY, vol. 14, no. 1, 83-97, January 1994) disclose the use of TCF-II or HGF for the treatment of hypoproteinemia, for the treatment of acute liver failure and for the treatment of acute renal failure.

The agent disclosed for preventing and/or treating multiple organ failure can be administered intravenously, intramuscularly or subcutaneously. These pharmaceutical preparation can be prepared according to a known pharmaceutical preparation method and, if necessary, pH conditioner, buffer and/or stabilizer can be added thereto. Dose of the present agent can be different depending on the severness of symptom, health conditions, age, body weight of a patient. Though the dose will not be restricted, pharmaceutical preparation comprising 0.6mg-600mg-TCF-II/day, preferably 6mg-60 mg-TCF-II/day, for one adult person can be administered at once or more. The dose of HGF can be nearly the same as that of TCF-II.

Administration as described above can prevent multiple organ failure caused by various kinds of mechanism described before or alleviate symptom thereof.

The present invention will be described below in detail by exemplifying examples 4-15.

### [Example 1]

### Purification of TCF-II

According to a method described in WO 90/10651 and a method of Higashio et al (Higashio, K. et. al, B.B.R.C., vol.170, pp397-404 (1990)), cell was cultured and purified TCF-II was obtained. That is, 3 x 10⁶ human fibroblast IMR-90 (ATCC CCL 186) cells were placed in a roller bottle containing 100 ml DMEM medium including 5% calf fetal serum and cultured by rotating it at the rate of 0.5-2 rpm for 7 days. When the total number of cell reached 1 x 10⁷ cells were deprived from the wall by trypsin digestion and collected at the bottom of bottle. And 100 g of ceramic with the size of 5-9 mesh (Toshiba Ceramic) was sterilized and put therein, which was cultured for 24hours. After then, 500 ml of the above culture medium was added thereto and the culture was continued. The total volume of culture medium was recovered every 7-10 days and fresh medium was supplemented. Production was kept for 2 months like this and 4 liters of culture medium was recovered per a roller bottle. Specific activity of TCF-II in culture medium obtained as above was 32 µg/ml. Culture medium (750 L) was concentrated by ultrafiltration using membrane filter (MW 6,000 cut; Amicon) and purified by 5-steps chromatography, that is, CM-Sephadex C-50 (Pharmacia), Con-A Sepharose(Pharmacia), Mono S column (Pharmacia), Heparin-Sepharose (Pharmacia) to yield purified TCF-IL This TCF-II had the same molecular weight and isoelectric point as described before.

### [Example 2]

### Production of recombinant TCF-II

According to the method described in WO 92/01053, cell transformed with TCF-II gene was cultured and purified TCF-II was obtained. That is, transformed Namalwa cell was cultured and 20 l of culture medium was obtained. This culture medium was treated by CM-Sephadex C-50 chromatography, Con-A Sepharose CL-6B chromatography and finally HPLC equipped with a Mono S column to yield about 11 mg of recombinant TCF-II. This TCF-II had also the same molecular weight and isoelectric point as described before.

### [Example 3]

### Production of recombinant HGF

Expression vector of HGFcDNA was constructed by inserting 2.4 kb fragment of transcription unit of mouse dihydrofolate reduce 7 tase (DHFR) into Nhel site of plasmid pcDNA1 and ,further, inserting 2.3 kb of HGFcDNA cloned by Miyazawa (BBRC 163, 967-973, 1989) into the downstream of Cytomegalovirus (CMV) promoter. The constructed HGFcDNA expression vector (µg and pSV2 neo 1 µg were cotransfected into Namalwa cell by liposome intervened transfection method using lipofectin. After transformed cells were screened by G418 resistency, gene amplification was carried out using methotrexate (MTX). HGF highly producing cell line was cultured in 2 L roller bottle containing 1 L DMEM medium including 5% bovine serum for 7 days. Culture was carried out using 20 roller bottles (2 rpm)and 21 L of culture medium was obtained. The culture medium obtained like this contained 4 mg/L HGF. According to a modified method of Higashio (Higashio et. al.,vol. 170, 397-404, 1990), 20 L of culturemedium containing HGF was purified by 3 steps chromatography, that is, CM-Sephadex C-50 (Pharmacia), MonoS column (Pharmacia) and Heparin 5-PW (Toso) and purified HGF with homogeneous mobility of SDS-electrophoresis was obtained in about 60 % yield.

### [Example 4]

### Protective effect on endotoxin-induced multiple organ failure

TCF-II (100 µg/mouse) obtained in example 2 was administered intravenously to 7 weeks old male ICR mice (25 mice/l group) twice daily for 5 days (only at the final day, once a day). The control group was treated with the vehicle (citric acid buffer solution with pH 6.03, hereinafter the same solution was used as control group). At 6 hours after the final administration, lethal dose of endotoxin (LPS-E-coli; 20mg/kg, Difco laboratories) was administered intravenously. The survival rates thereof was shown in figure 1. The survival rates on day 4 or later were 12% (3/25 mice) in the vehicle group, and those in the TCF-II group were 56% (14/25 mice).

From the results, TCF-II was confirmed to show an excellent protective effect on endotoxin-induced multiple organ failure.

### [Example 5]

### Protective effect on endotoxin-induced multiple organ failure

HGF (100 µ g/mouse) obtained in example 3 was administered intravenously to 7 weeks old male ICR mice (15 mice/l group) twice daily for 5 days (only at the final day, once a day). The control group was treated with the vehicle (citric acid buffer solution with pH 6.03). At 6 hours after the final administration lethal dose of endotoxin (LPS-E-coli; 20mg/kg, Difco laboratories) was administered intravenously. The survival rates thereof was shown in figure 2. The survival rates on day 4 or later were 13% (2/15 mice) in the vehicle group, and those in the 1mg/kg of HGF treated group was 33% (5/15 mice). From the results, HGF was confirmed to show an excellent protective effect on endotoxin-induced multiple organ failure.

### [Example 6]

### Protective effect on endotoxin-induced multiple organ failure

Animal model of multiple organ failure was made by continuously injecting endotoxin (LPS-E-coli; 10mg.kg/day, Difco, laboratories) to 6 weeks old male Wister rats using osmotic pump (Model 7 I2001, Alzet). After then, animals were divided into groups (9 rats/ 1 group) and vehicle or TCF-II (1 mg/kg) was administered intravenously once a day for 7 days. The results of clinical examination at the day after the final administration were shown in table 1. In the vehicle group, the serum levels of total protein, albumin, total cholesterol and the plasma levels of plasminogen were decreased at the day after the final administration, indicating that these rats were cachexia, but those in TCF-II treated group were significantly improved (Table 1). From the results, TCF-II was confirmed to show an excellent protective effect on multiple organ failure caused by endotoxin-induced cachexia.

**Table 1**

| Assay | Normal | LPS-induced model | |
|---|---|---|---|
| | | vehicle | TCF |
| Total protein (g/dl) | 5.4 ± 0.1 | 4.5 ± 0.1 | 5.5 ± 0.1** |
| Albumin (g/dl) | 2.5 ± 0.1 | 1.8 ± 0.1 | 2.4 ± 0.0** |
| Plasminogen(%) | 105.5 ±7.1 | 74.4 ± 3.6 | 96.6 ± 2.7** |
| Total cholesterol(mg/dl) | 73.2 ± 2.3 | 59.0 ± 3.6 | 88.6 ± 2.9** |

| | | | |
|---|---|---|---|
| (**: significant difference(p<0.01) from vehicle administered group) | | | |

### [example 7]

### Protective effect on dimethylnitrosamine-induced multiple organ failure

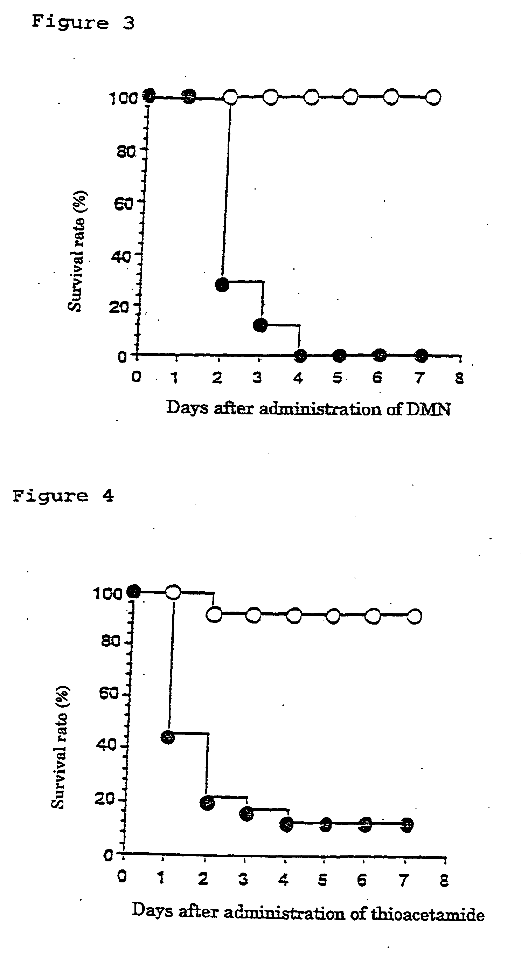

TCF-II (100 µg/mouse) was administered intravenously to 7 weeks old male ICR mice (25 mice/l group) twice daily for 5 days (only at the final day, once a day). The control group was treated with the vehicle. At 6 hours after the final administration, lethal dose of 0.15% dimethynitrosoamine (DMN) (vehicle:physiological saline solution, 0.1 ml/10 g body weight, Tokyo-kasei-kogyo) was administered intravenously. The results of clinical examination of mice at 24 hours after the onset was shown in table 2 and the the survival rates thereof was shown in figure 3. In the vehicle group, the plasma levels of GOT and GPT at 24hr after DMN administration were remarkably increased and the plasma clotting time was prolonged, but those of TCF-II treated group were significantly suppressed (Table 2). Further, in the vehicle group, all the mice died after 4 days, all the mice in the TCF-II group survived (Figure 3). From the results, TCF-II was confirmed to show an excellent protective effect on dimethylnitrosamine-induced multiple organ failure.

**Table 2**

| Assay | Normal | DMN-induced model | |
|---|---|---|---|
| | | vehicle | TCF-II |
| GOT(U/L) | 42±2 | 810±252 | 51±8** |
| GPT(U7L) | 28±3 | 1580±506 | 97±21** |
| Plasma clotting time(sec) | 17±0.0 | 22±2.2 | 17±0.1** |

| | | | |
|---|---|---|---|
| (**: significant difference (p<0.01) from vehicle group) | | | |

### [Example 8]

### Protective effect on drug intoxication-induced multiple organ failure

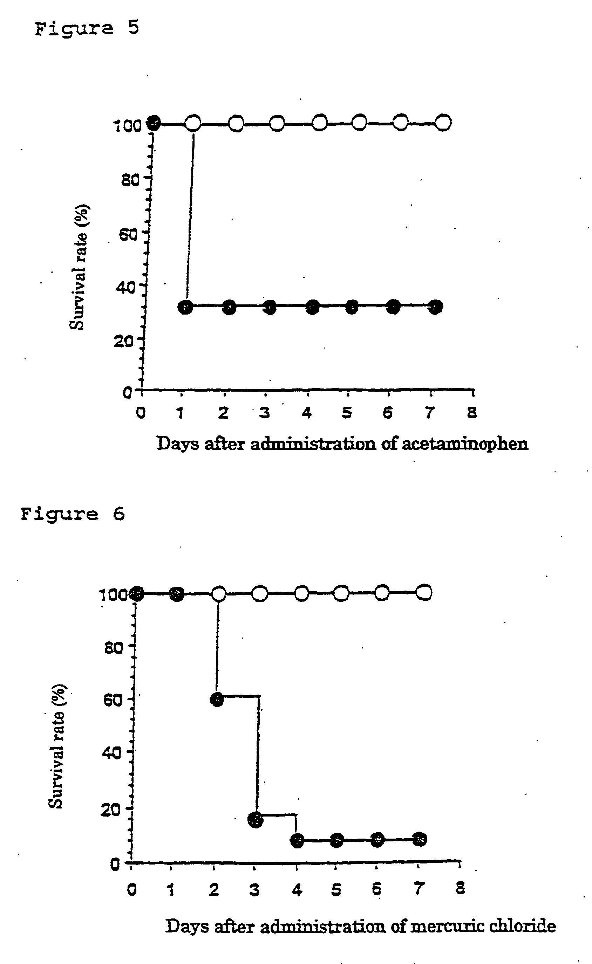

TCF-II (100 µg/mouse) was administered intravenously to 7 weeks old male ICR mice (25 mice/1 group) twice daily for 5 days (only at the final day, once a day). The control group was treated with the vehicle. At 6 hours after the final administration, lethal dose of thioacetamide (600 mg/kg, Wako-junyaku) or acetaminophen (800 mg/kg, Sigma) was administered. The survival rates thereof was shown in figure 4 and figure 5. In thioacetamide examination, survival rates after day 4 or later of vehicle administered group was 12% (3/25 mice), that of TCF-II administered group was 93% (23/25 mice). In acetaminophen experiment, though 68% (17/25 mice) of vehicle administered group died at the day after acetaminophen treatment, the whole mice of TCF-II administered group survived. From the results, TCF-II was confirmed to show an excellent protective effect on drug intoxication-induced multiple organ failure.

### [Example 9]

### Protective effect on multiple organ failure caused by mercuric chloride-induced renal insufficiency.

TCF-II (100 µg/mouse) was administered intravenously to 7 weeks old male ICR mice (25 mice/l group) twice daily for 5 days (only at the final day, once a day). The control group was treated with the vehicle. At 6 hours after the final administration, lethal dose of mercuric chloride (Wako-junyaku) was administered intravenously. The survival rates thereof was shown in figure 6. Though the survival rates after 4 days of vehicle administered group was 8% (2/25 mice), the whole mice of TCF-II administered group survived.

From the results, TCF-II was confirmed to show an excellent protective effect on multiple organ failure caused by mercuric chloride-induced.

### [Example 10]

### Protective effect on trypsin-induced multiple organ failure

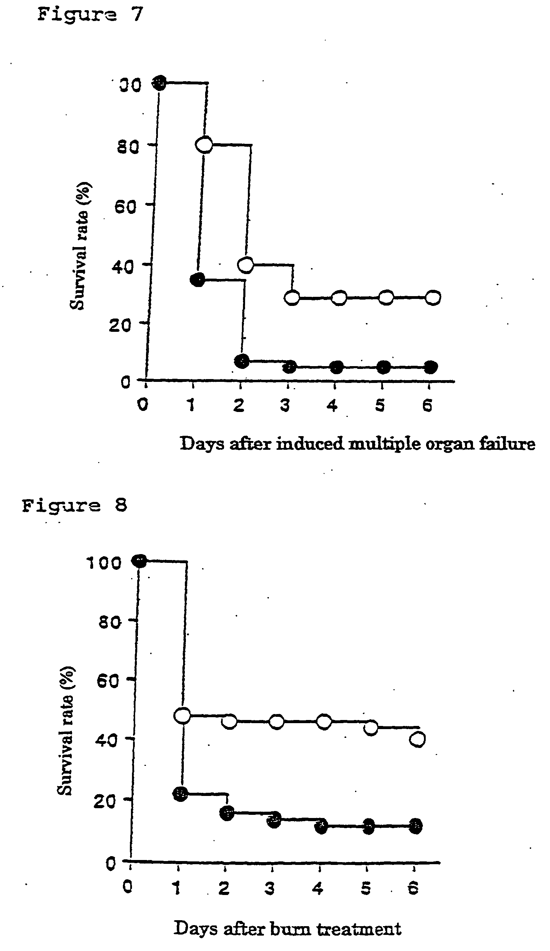

Vehicle (55 rats/1 group) or 1 mg/kg TCF-II (35 rats/1 group) was administered intravenously to 8 weeks old male Wister rats twice daily for 5 days (10 times). At the day after the final administration, 0.16 ml of mixed solution of lethal dose of trypsin (Sigma; 50000 U/ml) and taurocolic acid (Sanko-junyaku; 100 mg/ml) was injected into pancreas through the common bile duct. The survival rates thereof was shown in figure 7. Though the survival rates after 6 days of vehicle administered group was 5% (3/55 rats), that of TCF-II administered group was 29% (10/35 rats) (figure 7). From the results, TCF-II was confirmed to show an excellent protective effect on trypsin-induced multiple organ failure.

### [Example 11]

### Protective effect on burn-induced multiple organ failure

Vehicle or 1 mg/kg TCF-II was administered intravenously to 7 weeks old male Wister rats (50 rats/ 1 group) twice daily for 6 days (only once a day on the final day). At 6 hours after the final administration, 25% burn (250 °C, 30 sec.) was made on shaved back with a heating plate (Iwaki-glass). The survival rates thereof was shown in figure 8. And the results of clinical examination at 4 hours after burn treatment was shown in table 3. Decrease of circulating volume of Plasma (increase in Ht value, decrease in total protein, decrease in albumin) and hepatic derangement were observed and onset of multiple organ failure caused by burn shock was confirmed (Table 3). In addition, though the survival rates after 6 days of vehicle administered group was 12% (6/50 rats), that of TCF-II administered group was 40% (20/55 rats) (Figure 8). From the results, TCF-II was confirmed to show an excellent protective effect on burn-induced multiple organ failure.

**Table 3**

| | Before burn treatment | 4 hours after burn treatment |
|---|---|---|
| Hematocrit value (%) | 44.8±1.8 | 53.9±3.6 |
| Total protein (g/dl) | 7.2±0.5 | 5.8±0.7 |
| Albumin (g/dl) | 3.1±0.2 | 2.4±0.3 |
| GPT(U/L) | 20.5±5.8 | 150.0±30.4 |
| Urea nitrogen (mg/dl) | 215±1.9 | 43.5±7.3 |

### [Example 12]

### Protective effect on burn-induced multiple organ failure

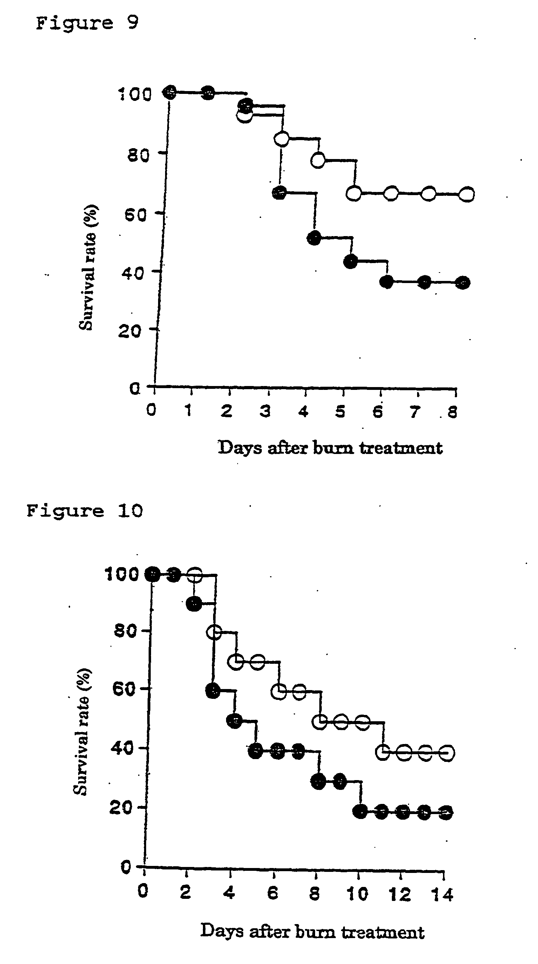

In 9 weeks old male Wister rats, 40% burn was made using 85°C hot water. After burn, rats were divided into 2 groups consisting of 27 rats each. Vehicle or 1 mg/kg TCF-II was administered intravenously 3 times/daily for 3 days (9 times). The survival rates thereof was shown in figure 9. Though the survival rates after 8 days of vehicle administered group was 37% (10/27 rats), that of TCF-II administered group was 67% (18/27 rats). From the results, TCF-II was confirmed to show an excellent protective effect on burn-induced multiple organ failure.

### [Example 13]

### Protective effect on burn-induced multiple organ failure

In 9 weeks old male Wister rats, 40% burn was made using 85 °C hot water. After burn, rats were divided into 2 groups consisting of 10 rats each. Vehicle or 1 mg/kg HGF was administered intravenously 3 times/daily for 3 days (9 times). The survival rates thereof was shown in figure 10. Though the survival rates after 11 days of vehicle administered group was 20% (2/10 rats), that of HGF administered group was 40% (4/10 rats). From the results, HGF was confirmed to show an excellent protective effect on burn-induced multiple organ failure.

### [Example 14]

### Manufacturing of pharmaceutical preparation of TCF-II

An example of manufacturing injections of recombinant TCF-II obtained example 2 was shown.
(1)

| | |
|---|---|
| TCF-II | 20 µg |
| human serum albumin | 100 mg |

The above composition was dissolved in citric acid buffer solution with pH 6.03 (consisting of 10 mM sodium citrate, 0.3 M sodium chloride, 0.03% polysolbate) so that the total volume would be 20ml. Then it was divided into vials containing 2 ml each after sterilization and sealed after lyophilization.
(2)

| | |
|---|---|
| TCF-II | 40 µg |
| Tween 80 | 1 mg |
| human serum albumin | 100 mg |

The above composition was dissolved in physiological saline solution for injections so that the total volume would be 20ml. Then it was divided into vials containing 2 ml each after sterilization and sealed after lyophilization.
(3)

| | |
|---|---|
| TCF-II | 20 µg |
| Tween 80 | 2 mg |
| Sorbitol | 4 g |

The above composition was dissolved in citric acid buffer solution with pH 6.03 so that the total volume would be 20ml. Then it was divided into vials containing 2 ml each after sterilization and sealed after lyophilization.
(4)

| | |
|---|---|
| TCF-II | 40 µg |
| Tween 80 | 1 mg |
| Glycine | 2 g |

The above composition was dissolved in physiological saline solution for injections so that the total volume would be 20ml. Then it was divided into vials containing 2 ml each after sterilization and sealed after lyophilization. 7
(5)

| | |
|---|---|
| TCF-II | 40 µg |
| Tween 80 | 1 mg |
| Solbitol | 2 g |
| Glycine | 1 g |

The above composition was dissolved in physiological saline solution for injections so that the total volume would be 20ml. Then it was divided into vials containing 2 ml each after sterilization and sealed after lyophilization.
(6)

| | |
|---|---|
| TCF-II | 20 µg |
| Sorbitol | 4 g |
| human serum albumin | 50 mg |

The above composition was dissolved in citric acid buffer solution with pH 6.03 so that the total volume would be 20ml. Then it was divided into vials containing 2 ml each after sterilization and sealed after lyophilization.
(7)

| | |
|---|---|
| TCF-II | 40 µg |
| Glycine | 2 g |
| human serum albumin | 50 mg |

The above composition was dissolved in physiological saline solution for injections so that the total volume would be 20ml. Then it was divided into vials containing 2 ml each after sterilization and sealed after lyophilization.
(8)

| | |
|---|---|
| TCF-II | 40 µg |
| human serum albumin | 50 mg |

The above composition was dissolved in citric acid buffer solution with pH 6.03 so that the total volume would be 20ml. Then it was divided into vials containing 2 ml each after sterilization and sealed after lyophilization.

### [Example 15]

### Manufacturing of pharmaceutical preparation of HGF

An example of manufacturing injections of recombinant HGF obtained in example 3 was shown.
(1)

| | |
|---|---|
| HGF | 40 µg |
| human serum albumin | 100 mg |

The above composition was dissolved in citric acid buffer solution with pH 6.03 so that the total volume would be 20ml. Then it was divided into vials containing 2 ml each after sterilization and sealed after lyophilization.
(2)

| | |
|---|---|
| HGF | 20 µg |
| Tween 80 | 1 mg |
| human serum albumin | 100 mg |

The above composition was dissolved in physiological saline solution for injections so that the total volume would be 20ml. Then it was divided into vials containing 2 ml each after sterilization and sealed after lyophilization.
(3)

| | |
|---|---|
| HGF | 30 µg |
| Sorbitol | 4 g |
| human serum albumin | 50 mg |

The above composition was dissolved in 0.01 M phosphate buffer solution with pH 7.0 so that the total volume would be 20ml. Then it was divided into vials containing 2 ml each after sterilization and sealed after lyophilization.

### Industrial applicability

The use of an agent in the manufacture of a medicament for preventing and/or treating multiple organ failure comprising TCF-II or HGF as an effective ingredient will be provided by the present invention.

The agent for preventing and/or treating multiple organ failure disclosed will be useful for preventing and/or treating the development from burn, disseminated intravascular coagulation (DIC), circulatory failure, hemorrhagic shock, infectious disease, acute pancreatitis, ischemic disorder, hepatorenal syndrome, gastrointestinal hemorrhage, nutritional metabolic failure, terminal cancer, acquired immunodeficiency syndrome (AIDS), deterioration of systemic conditions due to radiation affection and cachexia etc. to multiple organ failure.

## Claims

1. Use of Tumor cytotoxic factor-II (TCF-II) or Hepatocyte growth factor (HGF) in the manufacture of a medicament for the treatment or prevention of multiple organ failure.

2. Use according to claim 1 in which the multiple organ failure is induced by administration of chemical substances.

3. Use according to claim 2 in which the chemical substance is endotoxin.

4. Use according to claim 2 in which the chemical substance is dimethylnitrosamine.

5. Use according to claim 2 in which the chemical substance is trypsin.

6. Use according to claim 1 in which the multiple organ failure has drug intoxication as a primary cause.

7. Use according to claim 6 in which the drug is thioacetamide or acetaminophen.

8. Use according to claim 1 in which the multiple organ failure has renal insufficiency induced by mercuric chloride as a primary cause.

9. Use according to claim 1 in which the multiple organ failure is one induced by bum.

## Patentansprüche

1. Verwendung von tumorzytotoxischem Faktor-II (TCF-II) oder Hepatozytenwachstumsfaktor (HGF) zur Herstellung eines Medikaments zur Behandlung oder Prophylaxe von multiplem Organversagen.

2. Verwendung nach Anspruch 1, wobei multiples Organversagen durch die Verabreichung chemischer Substanzen ausgelöst wird.

3. Verwendung nach Anspruch 2, wobei die chemische Substanz Endotoxin ist.

4. Verwendung nach Anspruch 2, wobei die chemische Substanz Dimethylnitrosamin ist.

5. Verwendung nach Anspruch 2, wobei die chemische Substanz Trypsin ist.

6. Verwendung nach Anspruch 1, wobei das multiple Organversagen eine Arzneimittelvergiftung als Hauptursache hat.

7. Verwendung nach Anspruch 6, wobei das Arzneimittel Thioacetamid oder Acetaminophen ist.

8. Verwendung nach Anspruch 1, wobei das multiple Organversagen eine durch Quecksilberdichlorid ausgelöste Niereninsuffizienz als Hauptursache hat.

9. Verwendung nach Anspruch 1, wobei das multiple Organversagen durch eine Verbrennung ausgelöst wird.

## Revendications

1. Utilisation du facteur II cytotoxique tumoral (TCF-II) ou facteur de croissance des hépatocytes (HGF) dans la fabrication d'un médicament pour le traitement ou la prévention d'une défaillance d'organes multiples.

2. Utilisation selon la revendication 1, dans laquelle la défaillance d'organes multiples est induite par l'administration de substances chimiques.

3. Utilisation selon la revendication 2, dans laquelle la substance chimique est une endotoxine.

4. Utilisation selon la revendication 2, dans laquelle la substance chimique est la diméthylnitrosamine.

5. Utilisation selon la revendication 2, dans laquelle la substance chimique est la trypsine.

6. Utilisation selon la revendication 1, dans laquelle la défaillance d'organes multiples a une intoxication médicamenteuse comme cause principale.

7. Utilisation selon la revendication 6, dans laquelle le médicament est la thioacétamide ou l'acétaminophène.

8. Utilisation selon la revendication 1, dans laquelle la défaillance d'organes multiples a une insuffisance rénale induite par le chlorure mercurique comme cause principale.

9. Utilisation selon la revendication 1, dans laquelle la défaillance d'organes multiples est une défaillance induite par brûlure.
